# EUROPEAN PATENT APPLICATION

(11) **EP 1 774 963 A1**
(43) Date of publication of application: **18.04.2007**
(21) Application number: 06020895.6
(22) Date of filing: 05.10.2006
(51) Int. Cl.: A61K 9/14, A61K 36/28, A61P 15/14

(54) **Galactogenic compositions based on silymarin**

(30) Priority: 14.10.2005 IT MI20051942
(71) Applicant: S.I.I.T. S.r.l.-Servizio Internazionale Imballaggi Termosaldanti, 20090 Trezzano sul Naviglio MI (IT)
(72) Inventor: Di Pierro, Francesco, 20090 Trezzano sul Naviglio (MI) (IT)
(74) Representative: Minoja, Fabrizio

(57) **Abstract**

This invention relates to galactogenic compositions based on micronised silymarin.

## Description

### FIELD OF INVENTION

The present invention relates to galactogenic compositions containing micronised silymarin.

### INTRODUCTION

Milk constitutes a complete food for babies in both qualitative and energy-giving terms. It is an opaque liquid with an average specific gravity of 1030, an amphoteric or slightly alkaline reaction, a coefficient of viscosity of 1.41-2.56 at 37°C, and a sweetish taste; it contains fats in emulsion, sugars and mineral salts in solution, and protein substances in a particular state of pseudosolution.

Its protein concentration is 2.3%. The main protein is casein, which represents 50% of all the protein substances. The other proteins in milk are lactalbumin and lactoglobulin; however, the total quantity of both does not exceed that of casein. Milk contains all the essential aminoacids, including arginine, leucine, lysine, methionine and tryptophan.

Fats are found in milk in the form of a fine emulsion, appearing as small homogenous spheres called *milk globules.* Their average quantity is 1,026,000 per cu.mm. Their state of aggregation seems to be due to the presence of small amounts of casein on the periphery of the individual drops. The lipid content varies from one breast to the other, and one feed to another; the foremilk contains less fat than the hindmilk. Of the fatty acids contained in milk, the largest proportion is constituted by oleic acid, followed by palmitic acid.

Lactose constitutes the main proportion of the milk sugars, but glucose and a dextrin are also found in small amounts. Lactose also performs a mild laxative and diuretic action.

Minerals are represented by calcium, potassium, sodium, magnesium and phosphorus salts, whereas the iron content is negligible,

Milk also contains numerous proteolytic, lipolytic, glycolytic, peroxidase, catalase and reductase enzymes.

Milk contains several vitamins. It contains 200-600 IU per 100 ml of vitamin A, 10-15 IU of Vitamin B₁, 30-60 IU of riboflavin, and 5 mg per 100 mg of ascorbic acid, together with vitamins D, E and K.

Various antibodies can be passed to the baby through the milk, especially the agglutinins, as can various drugs which are administered to the mother, including salicylic and iodine compounds,

The breast processes milk from the constituents of the blood it is able to concentrate, and also performs synthesis processes on them. Thus milk proteins derive from plasma proteins, and lactose from blood glucose; lipids derive partly from the bloodstream, but another part is synthesised in the breast from small molecules.

The amount of milk secreted daily varies according to the period and the individual: on average, from 200 g on the 3rd day after the birth, increasing gradually to 1200-1500 cc towards the 6th month of lactation, after which it gradually declines.

The period of lactation in women corresponds to the functional activity of the breast: it consequently begins shortly before the birth, and ends at weaning. Leaving aside the initial period (the *colostrogenic* phase), the *lactogenic* phase begins some 48 hours after the birth, because during the early postpartum period the anterior pituitary gland is still under the inhibiting influence of the placental hormones which accumulate in considerable quantities during pregnancy, and therefore does not process prolactin. *Lactogenesis* does not take place until the 3rd or 4th day after the birth.

Total lack of milk secretion *(agalactia)* is exceptional: however, a particularly low secretion (hypogalactia) is sometimes observed in the first week after the birth, especially in primiparae. Women who eliminate less than 280 g of milk in a 24-hour period on the 5th day after the birth are classed as hypogalactic *(primary hypogalactia).*

*Secondary hypogalactia* appears after a period of normal milk secretion. Primary hypogalactia, which was very rare before 1950, has become progressively more frequent recently. Hypogalactia can be caused by a secretion defect due to delay in lactogenesis or to particular biological, neurohumoral or psychological conditions, especially in women who go out to work, by local or general disorders, or by stagnation of the secretion (caused by insufficient suction or an obstacle to emptying). In hypogalactia, a reduction in the blood lactose level is observed: in women with normal milk secretion the blood lactose level ranges between 0.30 and 0.80 g per thousand, whereas in hypogalactic women it is below 0.20 g per thousand. The treatment of hypogalactia is based mainly on identification and removal of the causal factor.

### PRIOR ART

No real pharmacological or natural treatment for hypogalactia has yet been found.

### TECHNICAL BACKGROUND

*Silybum marianum* (milk thistle) is a member of the Asteraceae family. It is an annual or biennial plant, found throughout the Mediterranean basin. The therapeutic use of this plant has been known since ancient times, and is even mentioned in the Bible. In later centuries, the plant was mentioned by Theophrastus (4th century B.C.), Pliny (1st century A.D.) and Dioscorus (1st century A.D.). However, it was not until c. 1500 that eminent doctors and experts in phytotherapy (including Mattioli) began to associate the virtues of this plant, especially the fruit and its derivatives, with the aspects that delineated its main field of use in later centuries, namely its effect on the liver.

Extract of *Silybum marianum* has proved clinically useful in numerous disorders, including acute and chronic hepatitis, hepatitis and cirrhosis induced by toxins or drugs, and alcoholic liver disease.

The principal constituent of *Silybum marianum* extract is silymarin, which is mainly found in the achenes of the plant and consists of a mixture of flavanolignans, the most representative of which are silibinin, isosilybin, silydianin and silychristin these products derive from the oxidative coupling of coniferyl alcohol (a lignan, the key building block for the construction of lignin) and taxifolin (a flavanonol).

Silymarin is not only well known and used as a liver protector, but on the basis of recent laboratory findings on animals also seems to have applications for preventing and treating some tumours (breast, prostate and melanoma).

The most active component of the mixture is silibinin, used to treat *Amanita phalloides* poisoning.

A recent study reported that silibinin bonds to P-glycoprotein (Pgp), which is involved in resistance to drugs by tumour cells and parasites like Leishmania Tropica. Pgp expels the drugs from the cells, reducing the intracellular accumulation of cytotoxic drugs (Maitrejan M., 2000). The action mechanism of silymarin involves:
- Inhibition of bonding between toxins and hepatocytes
- Reduction of glutathione oxidation and an increase in its level
- Stimulation of ribosomal RNA-polymerase with an increase in protein synthesis.

At molecular level, silymarin performs the following actions:
- Inhibition of lipid peroxidation in the hepatocytes and the ribosomal membranes
- Inhibition of damage to the DNA caused by peroxide and superoxide anion
- Inhibition of phosphodiesterase
- Inhibition of type III procollagen formation, and interference with the liver fibrosis process
- Inhibition of synthesis of the chemical mediators involved in inflammation (a-TNF, LTB4, PGE2)
- Modulator of P-glycoprotein.

The regenerating effect of silymarin on the hepatocytes seems to be selective for healthy cells, because silymarin does not promote the proliferation of hepatomas and other malignant cell lines (Takahara, 1986).

The pharmacokinetic data on silymarin show that the compound is not well absorbed after oral administration, with a bioavailability of only 23-47%, The peak plasma concentration is reached 2-4 hours after administration. The half-life is approximately six hours. Silymarin undergoes enteropathic circulation, as would be expected on the basis of its high molecular weight, and the concentration in the bile is many times greater than the serum concentration. Only 2-5% is excreted in the urine, whereas 20-40% is excreted in the bile in the form of glucuronide and sulphate. Phosphatidylcholine seems to increase the oral absorption of silymarin (Weyhenmeyer R.,1992).

### FIELD OF THE INVENTION

It has now been discovered that compositions based on micronised silymarin perform a strong galactogenic action, increasing milk production in breastfeeding women without altering the quality of their milk.

The bioavailability of silymarin as such is too low to exercise any galactogenic action, but it has been found that when silymarin is subjected to a particular micronisation technique which produces particles of under 20 microns, the pharmacokinetics, and consequently the plasma profile of the flavanolignans it contains, and their biological effect, are increased.

According to a preferred aspect, silymarin is subjected to micronisation according to a technique that produces particles of between 5 and 20 microns, which leads to a considerable improvement in the absorption kinetics, and consequently the biological action, of silymarin.

According to a particularly preferred aspect, the compositions according to the invention will contain micronised silymarin conjugated with phospholipids, as described in EP 0,209,038. Conjugation of silymarin with phospholipids gives a further increase in the plasma absorption peak.

According to a preferred aspect, silymarin will be present in the form of dried extract of *Silybum marianum* standardized to a 60% silymarin content.

A clinical trial was conducted on 3 groups of 25 breastfeeding women each. The first group was treated with 600 mg/day of micronised silymarin (5-20 microns of particulate matter) per os for 63 consecutive days, the second with non-micronised silymarin, and the third with a placebo.

It was observed that after 63 days, the quantity of milk produced by the women in the group treated with silymarin increased by an average of 517.32 g compared with day "0", namely an 85.94% increase. There was no increase in milk production in the group treated with non-micronised silymarin. In the group treated with the placebo, there was an increase of 170.20 g.

The biochemical parameters (water, fats, carbohydrates and proteins) remained within the physiological range in both groups.

The compositions according to the invention could be formulated suitably for oral administration, and prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

The compositions of the invention could also contain other ingredients with a complementary or otherwise useful activity, such as group B vitamins, vitamin C and vitamin E.

Some examples of formulations according to the invention are set out below.

### EXAMPLE 1 - 5.5 g sachets

| **INGREDIENT** | **Mg** | **%** |
|---|---|---|
| Micronised silymarin | 600.00 | 10.909 |
| Saccharose | 4480.00 | 81.455 |
| Anhydrous citric acid | 200.00 | 3.636 |
| Silicon dioxide | 10.00 | 0.182 |
| Sucrose ester | 30.00 | 0.545 |
| Flavour | 180.00 | 3.273 |
| **TOTAL** | **5500.000** | |

### EXAMPLE 2 - 5.5 g sachets

| **INGREDIENT** | **mg** | **%** |
|---|---|---|
| Micronised silymarin | 600.00 | 10.909 |
| Sorbitol | 4460.000 | 81.091 |
| Anhydrous citric acid | 200.00 | 3.636 |
| Silicon dioxide | 10.00 | 0.182 |
| Sucrose ester | 30.00 | 0.545 |
| Flavour | 180.00 | 3.273 |
| Acesulfame K | 20.000 | 0.364 |
| **TOTAL** | **5500.000** | |

### EXAMPLE 3 - 5.5 g sachets

| **INGREDIENT** | **mg** | **%** |
|---|---|---|
| Micronised silymarin | 600.00 | 10.909 |
| Isomalt | 4460.000 | 81.091 |
| Anhydrous citric acid | 200.00 | 3.636 |
| Silicon dioxide | 10.00 | 0.182 |
| Sucrose ester | 30.00 | 0.545 |
| Flavour | 180.00 | 3.273 |
| Acesulfame K | 20.000 | 0.364 |
| **TOTAL** | **5500.000** | |

### EXAMPLE 4 - 5.5 g sachets

| **INGREDIENT** | **mg** | **%** |
|---|---|---|
| Micronised silymarin | 600.00 | 10.909 |
| Vitamin C | 90.00 | 1.636 |
| Vitamin E | 30.00 | 0.545 |
| Saccharose | 4360.00 | 79.273 |
| Anhydrous citric acid | 200, 00 | 3.636 |
| Silicon dioxide | 10.00 | 0.182 |
| Sucrose ester | 30.00 | 0.545 |
| Flavour | 180.00 | 3.273 |
| **TOTAL** | **5500.000** | |

### EXAMPLE 5 - 5.5 g sachets

| **INGREDIENT** | **mg** | **%** |
|---|---|---|
| Micronised silymarin | 600.000 | 10.909 |
| Vitamin B 1 | 1.100 | 0.020 |
| Vitamin B2 | 1.700 | 0.031 |
| Vitamin B6 | 1.400 | 0.025 |
| Vitamin B12 | 0.0026 | 0.000 |
| Saccharose | 4475.7974 | 81.378 |
| Anhydrous citric acid | 200.000 | 3.636 |
| Silicon dioxide | 10.000 | 0.182 |
| Sucrose ester | 30.000 | 0.545 |
| Flavour | 180.000 | 3.273 |
| **TOTAL** | **5500.000** | |

### Example 6 - Format 0 capsules, 400 mg

| **INGREDIENT** | **mg** | **%** |
|---|---|---|
| Micronised silymarin | 150.000 | 37.500 |
| Microcrystalline cellulose | 83.000 | 20.750 |
| Dicalcium phosphate | 60.000 | 15.000 |
| Silicon dioxide | 6.000 | 1.500 |
| Magnesium stearate | 6.000 | 1.500 |
| *Gelatin shell* | *95* | |
| **TOTAL** | **400.000** | |

### Example 7 - Format 0 capsules, 400 mg

| **INGREDIENT** | **mg** | **%** |
|---|---|---|
| Micronised silymarin | 150.000 | 37.500 |
| Vitamin C | 22.50 | 5.625 |
| Vitamin E | 7.50 | 1.875 |
| Microcrystalline cellulose | 53.000 | 13.250 |
| Dicalcium phosphate | 60,000 | 15.000 |
| Silicon dioxide | 6.000 | 1.500 |
| Magnesium stearate | 6.400 | 1.500 |
| *Gelatin shell* | *95* | |
| **TOTAL** | **400.000** | |

### Example 8 - Format 0 capsules, 400 mg

| **INGREDIENT** | **mg** | **%** |
|---|---|---|
| Micronised silymarin | 150.000 | 37.500 |
| Vitamin B1 | 0.275 | 0.069 |
| Vitamin B2 | 0.425 | 0.106 |
| Vitamin B6 | 0.350 | 0.088 |
| Vitamin B12 | 0.0015 | 0.000 |
| Microcrystalline cellulose | 81.9485 | 20.487 |
| Dicalcium phosphate | 60.000 | 15.000 |
| Silicon dioxide | 6.000 | 1.500 |
| Magnesium stearate | 6.000 | 1.500 |
| *Gelatin shell* | *95* | |
| **TOTAL** | **400.0000** | |

### Example 9-1150 mg film-coated tablets

| **INGREDIENT** | **mg** | **%** |
|---|---|---|
| Micronised silymarin | 600.000 | 52.174 |
| Dibasic calcium phosphate | 280.000 | 24.348 |
| Microcrystalline cellulose | 207.800 | 18.070 |
| Croscarmellose sodium | 14.000 | 1.217 |
| Hydroxypropyl methylcellulose | 14.000 | 1.217 |
| Magnesium stearate | 10.000 | 0.870 |
| Silicon dioxide | 10.000 | 0.870 |
| Shellac | 8.000 | 0.696 |
| Titanium dioxide | 6.000 | 0.522 |
| E110 colouring | 0.200 | 0.017 |
| **TOTAL** | **1150.000** | **100.000** |

### Example 10 - 1200 mg film-coated tablets

| **INGREDIENT** | **mg** | **%** |
|---|---|---|
| Micronised silymarin | 600.000 | 50.000 |
| Vitamin C | 90.00 | 7.500 |
| Vitamin E | 30.00 | 2.500 |
| Dibasic calcium phosphate | 280.000 | 23.333 |
| Microcrystalline cellulose | 137.800 | 11.483 |
| Croscarmellose sodium | 14.000 | 1.167 |
| Hydroxypropyl methylcellulose | 14.000 | 1.167. |
| Magnesium stearate | 10.000 | 0.833 |
| Silicon dioxide | 10.000 | 0.833 |
| Shellac | 8.000 | 0.667 |
| Titanium dioxide | 6.000 | 0.500 |
| E110 colouring | 0.200 | 0.017 |
| **TOTAL** | **1200.000** | **100.000** |

### Example 11 -1100 mg film-coated tablets

| **INGREDIENT** | **mg** | **%** |
|---|---|---|
| Micronised silymarin | 600.000 | 54.545 |
| Vitamin B 1 | 1.100 | 0.100 |
| Vitamin B2 | 1.700 | 0.155 |
| Vitamin B6 | 1.400 | 0.127 |
| Vitamin B12 | 0.0026 | 0.000 |
| Dibasic calcium phosphate | 280.000 | 25.455 |
| Microcrystalline cellulose | 153.5974 | 13.963 |
| Croscarmellose sodium | 14.000 | 1.273 |
| Hydroxypropyl methylcellulose | 14.000 | 1.273 |
| Magnesium stearate | 10.000 | 0.909 |
| Silicon dioxide | 10.000 | 0.909 |
| Shellac | 8.000 | 0.727 |
| Titanium dioxide | 6.000 | 0.545 |
| E110 colouring | 0.200 | 0.018 |
| **TOTAL** | **1100.0000** | **100.000** |

### Example 12 - 900 mg retard tablets

| **INGREDIENT** | **mg** | **%** |
|---|---|---|
| Micronised silymarin | 300.000 | 33.333 |
| Dibasic calcium phosphate | 285.400 | 31.711 |
| Hydroxypropyl methylcellulose | 180.400 | 20.044 |
| Microcrystalline cellulose | 100.000 | 11.111 |
| Magnesium stearate | 10.000 | 1.111 |
| Silicon dioxide | 10.000 | 1.111 |
| Shellac | 8.000 | 0.889 |
| Titanium dioxide | 6.000 | 0.667 |
| E110 colouring | 0.200 | 0.022 |
| **TOTAL** | **900.000** | 100.000 |

### Example 13 - 1150 mg film-coated tablets

| **INGREDIENT** | **mg** | **%** |
|---|---|---|
| Micronised silymarin | 600.000 | 52.174 |
| Dibasic calcium phosphate | 280.000 | 24.348 |
| Microcrystalline cellulose | 172.262 | 14.979 |
| Croscarmellose sodium | 14.000 | 1.217 |
| Hydroxypropyl methylcellulose | 14.000 | 1.217 |
| Magnesium stearate | 10.000 | 0.870 |
| Silicon dioxide | 10.000 | 0.870 |
| Shellac | 40.000 | 3.478 |
| Glycerin | 3.747 | 0.326 |
| Talc | 2.510 | 0.218 |
| Lactose | 2.510 | 0.218 |
| Acetylated monoglycerides | 0.686 | 0.060 |
| Polyvinylpyrrolidone | 0.230 | 0.020 |
| E124 colouring | 0.055 | 0.005 |
| **TOTAL** | **1150.000** | 100.000 |

## Claims

1. Galactogenic compositions containing micronised silymarin.

2. Compositions as claimed in claim 1, wherein the micronised silymarin particles have dimensions of between 5 and 20 microns.

3. Compositions as claimed in claims 1 and 2, wherein silymarin is conjugated with phospholipids.

4. Compositions as claimed in the preceding claims, wherein silymarin is present in the form of dried extract of *Silybum marianum* standardized to a 60% silymarin concentration.

5. Use of micronised silymarin to prepare galactogenic compositions.
